# EUROPEAN PATENT APPLICATION

(11) **EP 1 074 245 A2**
(43) Date of publication of application: **07.02.2001**
(21) Application number: 00116679.2
(22) Date of filing: 02.08.2000
(51) Int. Cl.: A61K 7/48, A61P 7/10, A61P 21/00

(54) **Composition using mineral salts for therapeutic treatment**

(30) Priority: 06.08.1999 US 147634 P; 26.07.2000 US
(71) Applicant: Schwartz, Sam, Woodland Hills, Ca 91364 (US)
(72) Inventor: Schwartz, Sam, Woodland Hills, Ca 91364 (US)
(74) Representative: Petraz, Gilberto

(57) **Abstract**

A cosmetic and tissue cleansing composition including mineral salts, preferably Dead Sea mineral salts as an effective agent to minimize the effect of itching on the skin from a condition caused by psoriasis. In another form, the composition can be used as an exfoliating or scrubbing composition. The composition being formulated with a non-oily base which is water soluble such that the granules which are mineral salts act as exfoliating or scrubbing agents. The composition can also be formed for dental or mouth use. The composition can be used for therapeutic uses together with organic herbs and selectively aromatic essences.

## Description

### CROSS-REFERENCE TO RELATED APPLICATIONS

The present invention relates to U.S. Provisional Application Serial Number 60/072,477, filed January 26, 1998, and entitled "COSMETIC COMPOSITION"; U.S. Utility Application Serial No. 09/220,986, filed December 23, 1998, and entitled "COSMETIC AND TISSUE CLEANSING AND MOISTURIZING COMPOSITION"; and U.S. Provisional Application Serial Number 60/147,634, filed August 6, 1999, and entitled "COMPOSITION FOR USING MINERAL SALTS FOR THERAPEUTIC TREATMENT", the contents of which are incorporated by reference herein.

### BACKGROUND OF THE INVENTION

This application is directed to an improved cosmetic and cleansing composition.

Modern environmental conditions, such as heating and air conditioning, dryness, exposure to the sun, and environment pollution exert severe stress on the skin and accelerate the skin deterioration.

This can result in wrinkles, loss of firmness and elasticity, discoloration, dryness and other cosmetically undesirable effects. Additionally, medical conditions, such as psoriasis, can cause skin conditions which are exhibited as skin flaking and loss of skin tissue.

Although a number of cosmetic, cleansing and moisturizing compositions for use on the skin already exist, there is a need to provide a new cosmetic, cleansing and moisturizing composition which can provide advantages over those which are currently known.

It is also desirable to have compositions for use not only on humans but also on animals, as well as compositions that can be used within the mouth as a cleanser of the mouth or the teeth.

Different compositions are known using mineral salts from the Dead Sea, for instance, as bath salts.

It is not, however, known to use such minerals for other cosmetic or cleansing purposes and for purposes of use in the mouth.

Compositions using mineral salts with an oily based carrier are known. They however have disadvantages when used as a scrub or exfoliating cleansing composition. Such scrub and exfoliating compositions would be particularly useful on human skin and tissue.

A need exists for a new cosmetic and tissue cleansing and moisturizing composition.

### SUMMARY OF THE INVENTION

By this invention there is provided a cosmetic, cleansing and mouth formulations for use with human skin and on animal skin or tissue , which obviate the effect of modern environmental conditions on the human and animal body.

Some of these compositions include formulations suitable for entry into the mouth either as a dental toothpaste, cream or as a mouthwash.

An active ingredient of these compositions is a mineral salt, preferably Dead Sea salts.

The composition incorporates a new combination of ingredients particularly designed to provide protection from environmental pollution and to provide moisturization of the skin.

The cosmetic composition provides for tissue repair and protection for conditions caused by psoriasis.

The cosmetic composition simultaneously provides moisturization and control of oil when necessary.

In general, the composition according to one other aspect the present invention comprises: water and, emulsified and dispersed in the water, minerals salts, preferably Dead Sea salts.

One composition of the invention includes the use of salts, preferably mineral salts and more preferably, Dead Sea mineral salt in conjunction with other components, such as a lotion, to create a cleansing composition for use as an exfoliating and scrubbing composition for the body or face tissue of a human or animal.

The composition is prepared with a water soluble and moisturizing lotion while retaining the effective action of the Dead Sea mineral salt, which is preferably granules, such granules normally being water soluble.

In a preferred form of the invention, the composition include a moisturizing and/or non-oily base element for retaining the Dead Sea mineral salt granules in their granule form until used so that the mixture will act as an exfoliating scrub.

The non-oily base is water soluble and thereby permits easy removal without leaving an oily film on the skin. The effect however is to provide for moisturizing the skin.

The preferred composition includes a lotion which includes D.I. water, Methylparaben, Propylene glycol, Glycerin 96%, Steareth-2 (Lipocol S-2), Steareth-20 (Lipocol S-20), Cetyl Alcohol (Lipocol C), Stearyl Alcohol (Lipocol S), Safflower oil, Isopropyl myristate (Liponate IPM), DC 200 Fluid 100 cst., Propylparaben, Germail II and Fragrance at a low ratio, approximately 1:10, or one part lotion to about ten parts, there is added Dead Sea mineral salts. A minimum active amount, preferably the least amount of water is used in the formulation of the composition so that as part of the lotion the salt granules are not inclined break down.

Other scrubbing agents such as silica or pumice can selectively be the effective scrubbing agent or can be added to the mineral salt composition. The product can contain other scrubbing agents such as loofah or nut shells. An antibacteria agent can also be incorporated in the formula.

Other aspects of the invention include using the mineral bath salts with therapeutic herbs. Preferably, the Masada dead sea mineral bath salts are used with organic healing herbs. Aromatic essences can be added to these compositions. These compositions can relieve or assist in alleviating many common ailments.

### DETAILED DESCRIPTION OF THE PREFERRED EMBODIMENT

These and other features, aspects, and advantages of the present invention will become better understood with reference to the following description, appended claims.

The ingredients of a preferred embodiment of the present invention, their proportions, and the sequences of which they are a part for the purposes of mixing are described.

A new combination of ingredients results in a skin composition that provides protection against many types of skin damage, particularly itching and psoriasis, as well as other types of skin damage that can occur. Such other conditions can be flaking, redness, eczema, rashes or dry skin. Moreover, the composition is useful for cleansing the skin tissue, hands and the body and can be an effective scrubbing and exfoliating composition.

The composition of the present invention comprises base in which cosmetic components are mineral salts, preferably Dead Sea mineral salts. Another additive which is useful against itching is eugenol extract, namely an extract of cloves, and this can be added to the composition as required.

Typically, the composition of the present invention further comprises ancillary components such as: (1) a lipid-soluble component; (2) an emulsifier component; (3) an antioxidant component; (4) a preservative component; (5) a solvent component; (6) a thickener component; (7) a hydrophilic component; and (8) fragrance.

The ingredients included within these components are described in detail below.

The ingredients are dispersed in an emulsified composition by the method of preparation described below. "Dispersed" refers to any process by which the ingredients are uniformly distributed in the emulsified base, and includes dissolving, emulsifying, and forming a colloidal suspension.

### I. NATURE AND PROPORTION OF INGREDIENTS OF THE SKIN CREAM COMPOSITION

### A. The Cosmetic Components

Each of the cosmetic components, particularly the Dead Sea mineral salts, contributes to the improved properties of the cosmetic and cleansing composition of the present invention is present in a quantity sufficient to increase the smoothness, decrease the lumpiness, or decrease the itchiness, or edema of skin to which the composition is applied. Moreover, it reduces itching and repairs skin, and act as a cleansing agent.

An essential ingredient of the invention are mineral salts such as Dead Sea salts. These Dead Sea salts are salts obtained from the region of Israel or Jordan known as the Dead Sea, and which contain at least the following active ingredients:

| | | |
|---|---|---|
| Magnesium Chloride | (MgCl2) | 31.0 - 35.0% |
| Potassium Chloride | (Kcl) | 24.0 - 26.0% |
| Sodium Chloride | (NaCl) | 4.0 - 8.0% |
| Calcium Chloride | (CaCl2) | 0.4 - 0.6% |
| Magnesium Bromide | (MgBr2) | 0.3 - 0.6% |
| Sulphates | (SO4--) | 0.05 - 0.2% |
| Insolubles | | 0.05 - 0.3% |
| Water of Crystallization | | 34.0 - 38.0% |

### B. The Ancillary Components

The composition of the present invention can further comprise a lipid-soluble component to provide added smoothness. The lipid-soluble component can comprise at least one ingredient selected from the group consisting of: (1) dimethicone; (2) bisabolol; (3) polyoxyethylene fatty acid esters; (4) cetyl alcohol; (5) a glyceryl triester of a medium-chain carboxylic acid selected from the group consisting of tricaproin, tricaprylin, tricaprin, and mixtures thereof; (6) white petrolatum; and (7) mineral oil.

The ancillary components, whose use is optional but preferable, impart additional desirable properties to the skin cream composition of the present invention. These components can include: (1) a lipid-soluble component; (2) an emulsifier component; (3) an antioxidant component; (4) a preservative component; (5) a solvent component; (6) a thickener component; (7) a hydrophilic component; and (8) fragrance. Preferably, the composition of the present invention comprises all the ancillary components as indicated below:

As comprised in a skin cream, the composition with the solvent provides for greater uniformity and ease of preparation. The solvent component can include one or two ingredients.

The thickener component improves the flow and rheological properties of the cosmetic composition. It permits the composition to be retained in a formulated state when applied to the skin. One or more ingredients can comprise the thickener component. The hydrophilic component can comprise one or multiple ingredients.

If necessary, a preservative component can be used to retard microbial and mold growth in the composition. The preservative component can also act as a stabilizer. The lipid-soluble component can also comprise one or multiple ingredients, and these can be varied depending upon the cosmetic composition as to be used. For instance, the composition can be formulated for normal skin, dry skin, or oily skin, and different lipid components can be used under those conditions.

### 1. The Lipid-Soluble Component

The cosmetic composition can comprise the additional ancillary ingredients whose use is optional but preferable. These ancillary ingredients can include a solvent component, a preservative component, a thickener component, a hydrophilic component, a lipid-soluble component and a pigment. As necessary or suitable, a fragrant component can also be added. Various combinations of these ingredients can be used as the solvent, lipid, thickener or hydrophilic component.

The glyceryl triester of the medium-chain carboxylic acid can be tricaprylin. The lipid-soluble component comprises dimethicone, bisabolol, polyoxyethylene fatty acid esters, cetyl alcohol, tricaprylin, white petrolatum, and mineral oil.
The dimethicone can comprise about 0.75% of the composition, the bisabolol comprises about 0.1% of the composition, the polyoxyethylene fatty acid esters comprise about 2.5% of the composition, the cetyl alcohol comprises about 4% of the composition, the tricaprylin comprises about 7.5% of the composition, the white petrolatum comprises about 3% of the composition, and the mineral oil comprises about 6.0% of the composition.

### 2. The Emulsifier Component

The skin cream composition of the present invention can further comprise an emulsifier component. Emulsifiers serve two functions. They act like a solubilizing agent to combine the water-soluble and non-water-soluble phases together; that is, to form a stable bridge between the waters and the oils of the ingredients. The emulsifiers also serve as emollients, providing a pleasant, esthetically appropriate tactile feeling when the emulsified composition is applied to the skin. The emulsifier component is present in a quantity sufficient to combine water-soluble and non-water-soluble phases of the composition.

The emulsifier component can comprise at least one of a mixture of mono- and distearate esters of polyoxyethylene and free polyethylene oxide, partial esters of lauric, palmitic, stearic, and oleic acids and hexitol anhydrides, and 120-mole ethoxylated jojoba oil. The emulsifier component can comprise a mixture of mono- and distearate esters of polyoxyethylene and free polyethylene oxide, partial esters of lauric, palmitic, stearic, and oleic acids and hexitol anhydrides and 120-mole ethoxylated jojoba oil.

Preferably, the mixture of mono- and distearate esters of polyoxyethylene and free polyethylene oxide comprises about 1.25% of the composition, the partial esters of lauric, palmitic, stearic, and oleic acids and hexitol anhydrides comprise about 0.13% of the composition, and the 120-mole ethoxylated jojoba oil comprises about 1.0% of the composition.

### 3. The Antioxidant Component

The composition according to the present invention can further comprise an antioxidant component. The antioxidant component prevents oxidation of the ingredients of the composition. The antioxidant component can be a mixture of 70% propylene glycol, 20% propyl gallate, and 10% citric acid. The antioxidant component comprises about 0.0011% of the composition.

### 4. The Preservative Component

The skin cream composition according to the present invention can further comprise a preservative component. The preservative component is used to prevent the growth of microbes in the emulsified skin cream composition, which is typically manufactured under clean, but non-sterile conditions. The preservative component can comprise at least one of imidazolyl urea and a complex of propylene glycol, phenoxyethanol, chlorphenesin and methylparaben.

The preservative component can comprises both imidazolyl urea and a complex of propylene glycol, phenoxyethanol, chlorphenesin and methylparaben, the imidazolyl urea comprises about 0.0011% of the composition, and the complex of propylene glycol, phenoxyethanol, chlorphenesin and methylparaben comprises about 2.5% of the composition.

In the complex of propylene glycol, phenoxyethanol, chlorphenesin and methylparaben, the propylene glycol comprises from about 30% to about 45% of the complex, the phenoxyethanol comprises from about 22% to about 37% of the complex, the chlorphenesin comprises from about 11% to about 22% of the complex, and the methylparaben comprises from about 11% to about 22% of the complex.

### 5. The Solvent Component

The composition according to the present invention can further comprise a solvent component. The use of a solvent component allows greater uniformity and ease of preparation. The solvent component can include at least one ingredient selected from the group consisting of ethylene glycol, propylene glycol, and 1,3-butylene glycol. The solvent component can comprise 1,3-butylene glycol and the solvent component comprises about 4.0% of the composition.

### 6. The Thickener Component

The composition according to the present invention can comprise a thickener component in a quantity sufficient to retain the composition when it is applied to the skin of a wearer. The thickener component can comprise at least one ingredient selected from the group consisting of alginate derivatives and preneutralized carbomer 430. The skin cream composition can comprise both alginate derivatives and preneutralized carbomer 430. The alginate derivative can comprise about 0.2% of the composition, and the preneutralized carbomer 430 and be about 0.5% of the composition.

### 7. The Hydrophilic Component

A composition according to the present invention can further comprise a hydrophilic component. The hydrophilic component can comprise a polar complex consisting essentially of mannitol, arginine, serine, pyrrolidone carboxylate, sucrose, citrulline, glycogen, histidine, alanine, threonine, glutamic acid, and lysine. The polar complex can comprise about 1.0% of the composition.

### 8. Fragrance

The composition according to the present invention can further comprise fragrance. The use of fragrance is well known in the cosmetic art, and need not be described further. The fragrance can comprise about 0.45% of the composition, although this can vary depending upon the fragrance used.

Compositions according to the present invention can further comprise other components used in the cosmetic art, such as pigments and other conventional excipients. The use of such ingredients is well known in the cosmetic and cleansing art and need not be described further here. The pigment component gives the cosmetic composition an aesthetically desirable appearance and different pigments can be used as variables in relation to the skin tone of the intended user.

Other components of the composition which permit for its application to the skin include propylene, glycol, and effective amounts of aloe vera juice, sunflower oil, kessco OP, liponate, other hydrogenated vegetable oil, lipo GMS, stearic acid, and lipomulse. As necessary, the product could also include, when intended for application to the skin, a suitable sunscreen composition. In different forms, although aloe vera juice is considered, other extracts are possible. This could, for instance, be extracts from other fruits such as balsalm or apricot.

An example of the cosmetic composition is set out as follows, together with the relative percentages of content, and also an indication of the relative phase of those products. The relationship of the phase is also set out in regard to the method of formulating the composition:

### SAMPLE COMPOSITION FOR COSMETIC CLEANSING AND MOISTURIZING PRODUCT

| PHASE | % | MATERIAL |
|---|---|---|
| 1 | 64.9500 | DEIONIZED WATER |
| | 1.000 | ALOE VERA JUICE |
| | 0.2000 | METHYLPARABEN |
| 2 | 3.0000 | PROPYLENE GLYCOL |
| | 0.1000 | KELTROLT |
| 3 | 1.0000 | SUNFLOWER OIL |
| | 2.0000 | KESSCO OP |
| | 2.0000 | LIPONATE GC |
| | 1.0000 | THODORSIL 47V 350/SF 96-350 |
| | 2.0000 | HYDROGENATED VEGETABLE OIL |
| | 3.5000 | LIPO GMS 470 - WITCONOL 2407 |
| | 2.0000 | STEARIC ACID |
| | 2.0000 | LIPOMULSE 165/ARLACEL 165V |
| | 1.0000 | LIPOCOL C/ALFOL 16 |
| | 1.0000 | LIPO WAX D |
| | 0.1000 | PROPYLPARABEN |
| | 0.1000 | VITAMIN E ACETATE |
| 4. | 0.5000 | EUGENOL EXTRA USP |
| | 0.4000 | MACKSTAT DM |
| | 0.0500 | WHITE DIAMOND B/M63149 |
| | 0.1000 | COVIOX T70 |
| 5 | 10.0000 | DEIONIZED WATER |
| | 5.0000 | DEAD SEA SALT |
| TOTAL | 103.0000 | |

The method of formulation of such a sample is as follows:

| STEP | TEMP | |
|---|---|---|
| 1 | 80° | PHASE 1 |
| | | Into a main SS compounding tank, meter Dionized Water. Add Aloe Vera. Turn on mixer. Begin heating to 80°C. |
| 2 | 70° | When the temperature of the main tank is at 70°C sprinkle slowly Methylparaben. Mix well until completely dissolved. Recirculate batch to insure that no solid is left at the bottom. |
| 3 | RT | PHASE 2 |
| | | Into a suitably size container, add Phase 2 ingredients. Mix well into a uniform slurry. |
| 4 | 80° | When the temperature of the main tank is at 80°C add slowly Phase 2 slurry. Note: Be sure to mix Phase 2 slurry well before adding to Phase 1. Keltrol T has a tendency to settle at the bottom. |
| 5 | 80° | PHASE 3 |
| | | Into a separate ss auxiliary tank, add Phase 3 ingredient except Propylparaben. Begin heating to 80°CX. Turn on mixer when most of the waxes are melted. |
| 6 | 70° | When temperature of Phase 3 is at 70°C sprinkle in slowly Propylparaben. Be sure to evacuate valve to insure that no solids are entrapped. |
| 7 | 80° | When the temperature of Phase 3 is at 80°C and ready to be added to Phase 1, add Vitamin E Acetate. |
| 8 | 80° | EMULSIFICATION |
| | | When both Phase 1 and Phase 3 reach 80°C, add Phase 3 to main batch. Mix well for 15 minutes. Recirculate batch on and off. |
| 9 | | Begin cooling. |
| 10 | 38° | PHASE 4 |
| | | When temperature of the main batch is at 38°C add Phase 4 ingredients. Mix well. |
| 11 | RT | PHASE 5 |
| | | Into a suitably sized container add Phase 5 ingredients. Mix well until completely dissolved. |
| 12 | 38° | When the temperature of the main batch is 38° add the solution of Phase 5. Mix well. |
| 13 | 30° | Discontinue mixing and cooling at 30°C |

### SAMPLE SCRUBBING OR EXFOLIATING COMPOSITION

Such a composition would include the following ingredients:

| MATERIAL | % |
|---|---|
| D.I. water | 75.28 |
| Methylparaben | 0.25 |
| Propylene glycol | 2.00 |
| Glycerin 96% | 5.00 |
| Steareth 2 (Lipocol S-2) | 1.00 |
| Steareth-20 (Lipocol S-20) | 2.00 |
| Cetyl Alcohol (Lipocol C) | 1.50 |
| Stearyl alcohol (Lipocol S) | 1.50 |
| Safflower oil | 4.00 |
| Isopropyl myristate (Liponate IPM) | 5.00 |
| DC 200 Fluid 11 cst. | 1.00 |
| Propylparaben | 0.07 |
| Germail II | 0.20 |
| Fragrance | 0.20 |

Additionally, there is Dead Sea salts added in a range of about 1:10, namely ten parts salt to one part of lotion. As needed, other scrubbing agents such as pumice or silica can be added.

Although the invention has been described with regard to a cosmetic product related to soothing the effects of psoriasis, there are other formulations of the product which essentially includes mineral salts, preferably Dead Sea salt which can be used for different purposes. As such, a mouthwash formulation can be provided. The product will assist in combatting bacteria and gum irritation and inflammation. The product can moisturize and assist in nourishment of the skin.

The Dead Sea salts can be formulated with an amount of water and a suitable fragrance for these purposes. Likewise, as a shampoo product, the formulation can be prepared for use on human hair as well as animal hair. An essential ingredient of such a product is also mineral salts, such as Dead Sea mineral salts, and the percentage of Dead Sea salt can vary according to the purpose and formulation.

### CONDITIONING SHAMPOO COMPOSITION

| TSP | % |
|---|---|
| Masada salts | 2.00 |
| DI water | 57.50 |
| Cocomido propyl betaine/(Norfox) | 7.00 |
| SLES-2/(Norfox)(Sodium laureth sulfate) | 20.00 |
| SLES-60/(Norfox) | 5.00 |
| Cocomido propyl amine oxide/Schercamox CAA-G (Scher) | 2.50 |
| Crodafos SG (Croda) (PTG-5 Ceteth-10 phosphate) | 1.00 |
| Oleamide DEA/Schercomid ODA (Scher) | 2.50 |
| Tritisol/sol wheat protein (Croda) (Hydrolyzed wheat protein) | 0.70 |
| Aloe 10:1(D-D Chem) | 0.20 |
| Neutral henna | 0.10 |
| Algae extract | 0.10 |
| Comfrey leaf | 0.10 |
| Goldenscal root | 0.10 |
| Elderflower | 0.10 |
| Nettle | 0.10 |
| Rosemary | 0.10 |
| Red clover | 0.10 |
| panthenol (Roche) | 0.10 |
| DMDM hydantoin | 0.40 |
| Fragrance (Custom Essence) CE-22601 coal tar mask | 0.30 |
| TOTAL | 100.00 |

The procedure for mixing the formulation is as follows:
1. Add ingredients of phase A individually with mixing. Mix until homogenous.
2. When phase A is homogeneous, add individually ingredients of phase B. Note: If scale up testing indicates that the fragrance is clouding the product, premix 1:1 with Triton X-100.
3. Adjust pH to around 6.

Further, the different compositions and formulations can also be used, for instance, in the soothing of the skin when subjected to acne.

The composition is applied to the skin to stop itching. When applied to animals particularly it is useful against skin disorders, and joint or arthritic problems. It is particularly useful on dogs and horses.

Other forms of the invention includes those when applied in liquid form. It can also be applied as saturated wipe or as a compress. Moreover, it is applicable as a scrubbing or exfoliating composition. Additionally, it can be formulated as an inhalant product where another component is eucalyptus.

A different composition of the formula can be used for inhaling to ease nasal or sinus congestion and to soothe coughing irritations due to bronchitis or similar conditions.

### Therapeutic Compositions

Masada dead sea mineral bath salts can be used with organic healing herbs and, selectively, aromatic essences. These compositions can be used to stimulate circulation, relieve aches and pains, soften skin, promote relaxation, and draw out toxins from the body. With the salts fortified with the organic herbs and aromatic essences, many common ailments can be at least partly relieved. Different compositions are set out as follows:

| **MASADA MINERAL HERB SPA BATH SALTS TREATMENT COLLECTION** | | |
|---|---|---|
| **Therapy for** | **Therapeutic Organic Herbs** | **Aromatic Essences** |
| PMS RELIEF | Chasteberry & Dandelion Root | Lavender & Geranium |
| | Helps relieve aches, cramping; | Relaxes and calms sensitivities |
| | Eases excess water retention discomfort | Helps lift blue moods |
| COLDS & FLU | Echinacea, Goldenseal Root, Lemon Balm | Eucalyptus |
| | Helps strengthen the body's immune system | Helps relieve sinus congestion |
| | Eases flu aches and pains | Reduces inflammation |
| DETOX, M.D. | Green Tea, Milk Thistle Root, Ginseng Root, Rosemary & Juniper | Rosemary & Juniper |
| | | Combats mental fatigue |
| | Invigorates and fortifies the body | Enhances physical energy |
| | Draws out toxins, stimulating circulation | |
| TOTAL RELAXATION | Valerian, Kava Kava & Passion Flower | Lavender, Orange Blossom and Vetivyr |
| | Calms frayed nerves and helps promote sleep | |
| | Helps relieve tension and fatigue | Relaxes the mind & body |
| | Relaxes tense, aching muscles | Promotes peace & balance |
| SKIN CALMING | Calendula, Clove & Chamomile | Chamomile & Melissa & Linden |
| | Excellent for psoriasis and eczema | Soothes irritation and redness |
| | Relieves itchy, burning, irritated skin | Eases stress and tension |
| | Eases discomfort from allergies, insect bites | |
| | Clams and soothes swelling | |
| JOINT & MUSCLE RELIEF | Thyme, Juniper Berries & Yarrow Wintergreen, Camphor & Rosemary | Wintergreen |
| | | Warms stiff muscles and joints |
| | Excellent for arthritis and rheumatism | Eases tension |
| | Relaxes muscles and soothes cramps and aching joints | |

Although the selected organic herbs are set out in the examples, it is clear that many other herbs can be used in a manner commonly used for different ailments and conditions. The herbs may also be non-organically produced or grown herbs. These conditions can be applicable both for humans and for animals, and the therapeutic uses can be for humans and for animals. An advantage of this technique is that where a patient cannot normally take herbs orally, the herbs can be absorbed through the skin by these bath salts products which can be used for bathing or showering and the like.

The invention has been described with regard to several examples which are for illustrative purposes only.

## Claims

1. A composition comprising a mineral salt having as active ingredients Magnesium Chloride, Potassium Chloride, Sodium Chloride, water, a lipid soluble compound, oils, and a herbal component, the composition having components present in sufficient quantities to effectively minimize against a condition of at least one of bodily muscular cramp, excess water retention and swelling.

2. A composition according to claim 1, further comprising an aromatic essence.

3. A composition according to anyone of claims 1 and 2, comprising at least about 5% Dead Sea salts.

4. A composition according to anyone of the preceding claims, further comprising an organic herb component.

5. A composition according to anyone of the preceding claims, wherin said mineral salt is emulsified and dispersed in water.

6. A composition according to anyone of the preceding claims, wherein the herb component is selected to be at least one of chasteberry, dandelion root, echinacea, golden seal root, lemon balm, green tea, milk thistle root, ginseng root, rosemary, juniper, valerian, kava kava, passion flower, calendula, clove, chamomile, thyme, juniper berries, yarrow wintergreen, camphor or rosemary.

7. A composition according to anyone of the preceding claims, comprising an aromatic essence.

8. A composition according to claim 7, wherein the essence is at least one of lavender, geranium, eucalyptus, rosemary, juniper, lavender, orange blossom, vetivyr, chamomile, melissa, linden or wintergreen.

9. A composition according to anyone of the preceding claims, comprising a compound for application to the body as a bath salt during bathing or showering.
